# EUROPÄISCHE PATENTANMELDUNG

(11) **EP 2 204 130 A1**
(43) Veröffentlichungstag der Anmeldung: **07.07.2010**
(21) Anmeldenummer: 08106039.4
(22) Anmeldetag: 30.12.2008
(51) Int. Cl.: A61B 17/88, A61B 17/80

(54) **Schneidewerkzeug und Osteosynthese-Set**

(71) Anmelder: Medartis AG, 4057 Basel (CH)
(72) Erfinder: Langer, Barry, 4106 Therwil (CH); Ammann, Marc, 8004 Zürich (CH); Schlund, Balz, 8032 Zürich (CH); Röllinghoff, Micha, 4612 Wangen b. Olten (CH)
(74) Vertreter: Müller, Christoph Emanuel

(57) **Zusammenfassung**

Die Erfindung betrifft ein Schneidewerkzeug zum Durchtrennen von Knochenplatten (4), insbesondere ein Schneidezange (1). Das Schneidewerkzeug enthält mindestens eine erste Schneidekante (2) und mindestens eine zweite Schneidekante (3), mittels welcher die Knochenplatte (4) in mindestens einer Schneideposition (S) entlang einer Schnittfläche (5) durchtrennbar ist. Weiterhin umfasst das Schneidewerkzeug mindestens einen Niederhalter (6), welcher derart ausgebildet und angeordnet ist, dass beim Durchtrennen der Knochenplatte (4) eine Verbiegung der Knochenplatte (4) durch einen Kontakt des Niederhalters (6) mit der Knochenplatte (4) im Wesentlichen verhindert wird. Der Niederhalter (6) ist dabei derart ausgebildet und angeordnet, dass die Knochenplatte (4) in einer Einführrichtung (E) im Wesentlichen geradlinig in die Schneideposition (S) bringbar ist.

## Beschreibung

Die vorliegende Erfindung betrifft ein Schneidewerkzeug zum Durchtrennen von Knochenplatten sowie ein Osteosynthese-Set mit mindestens einer Knochenplatte und mindestens einem Schneidewerkzeug.

Knochenplatten, welche im Bereich der Osteosynthese verwendet werden, sind in einer grossen Vielfalt von Grössen und Formen erhältlich. Dennoch ist es für einen Chirurgen oftmals erforderlich, derartige Knochenplatten an die individuelle Anatomie eines Patienten oder an den konkret vorliegenden Knochenbruch oder -defekt anzupassen. Zu diesem Zweck werden vor allem intraoperativ Schneidewerkzeuge, beispielsweise Schneidezangen verwendet, um die Knochenplatten an einer geeigneten Stelle zu durchtrennen und damit ein Knochenplattenteil mit einer gewünschten Grösse und Form bereitzustellen.

Eine gattungsgemässe Schneidezange ist bereits in DE 43 08 319 offenbart. Die dort beschriebene Schneidezange weist zwei Griffhebel mit jeweils einer daran befestigten Schneidebacke auf. Beide Schneidebacken verfügen über jeweils eine Schneidekante. Eine zu durchtrennende Knochenplatte kann in eine Schneideposition gebracht werden, in welcher sie mittels der beiden Schneidenkanten entlang einer Schnittfläche durchtrennbar ist.

An einer der beiden Schneidebacken weist die Schneidezange eine Auflagefläche und einen Niederhalter auf, zwischen denen die Knochenplatte festgelegt werden kann, so dass beim Durchtrennen kein Verkanten oder Verformen der Knochenplatte eintritt. Darüber hinaus weist die Auflagefläche einen von ihr hervorragenden Haltestift auf, auf welchen eine Durchgangsöffnung der Knochenplatte, beispielsweise eine Schraubenöffnung, gesteckt werden kann, was zu einer weiteren Festlegung der Knochenplatte beim Durchtrennen führt.

Diese im Stand der Technik gezeigte Schneidezange weist jedoch eine Reihe von Nachteilen auf. Eine Knochenplatte muss zunächst mit einer Durchgangsöffnung auf den Haltestift aufgesetzt werden und anschliessend um diesen Haltestift herum in einen Zwischenraum zwischen der Auflagefläche und den Niederhalter geschwenkt werden, was recht aufwändig ist.

Darüber hinaus schränken die Anordnung und die Dimensionen des Haltestifts die Auswahl der mit dieser Schneidezange durchtrennbaren Knochenplatten erheblich ein. Einerseits muss nämlich der Innendurchmesser der Durchgangsöffnung auf den Aussendurchmesser des Haltestifts abgestimmt sein. Andererseits wird durch den Abstand des Haltestifts von der Schneidekante auch der Abstand zwischen Schnittfläche und Durchgangsöffnung der Knochenplatte festgelegt. Damit können beispielsweise keine Knochenplatten durchtrennt werden, bei denen der Abstand zweier benachbarter Durchgangsöffnungen dem Abstand zwischen Haltestift und Schneidekante entspricht; in diesem Falle würde beim Festlegen der einen Durchgangsöffnung am Haltestift die Umrandung der anderen Durchgangsöffnung durchtrennt und zwei Teilstege der Umrandung hinterlassen, welche von der Knochenplatte abstehen. Weiterhin kann der Haltestift eine allfällige Innenkontur der Durchgangsöffnung beschädigen. Zudem ist das Durchtrennen von Knochenplatten mit zwei oder mehreren in etwa parallelen Laschen, wie beispielsweise Y- oder n-förmigen Knochenplatten, erschwert oder gar nicht erst möglich, da einige der Laschen, welche nicht zum Durchtrennen vorgesehen sind, an Bauteilen der Schneidezange anstossen können.

Es ist daher eine Aufgabe der vorliegenden Erfindung, die Nachteile dieser bekannten Schneidezange zu überwinden und insbesondere ein Schneidewerkzeug bereitzustellen, welches ein Durchtrennen einer grösseren Vielfalt von Knochenplatten sowie ein leichteres und präziseres Ein- und/oder Ausführen der Knochenplatte in die Schneideposition erlaubt.

Diese und weitere Aufgaben werden durch ein Schneidewerkzeug und ein Osteosynthese-Set mit den Merkmalen der kennzeichnenden Teile der unabhängigen Patentansprüche gelöst.

Das erfindungsgemässe Schneidewerkzeug dient dem Durchtrennen von Knochenplatten. Beispielsweise kann es sich bei dem Schneidewerkzeug um eine Schneidezange handeln. Das Schneidewerkzeug enthält mindestens eine erste Schneidekante und mindestens eine zweite Schneidekante, mittels welcher die Knochenplatte in mindestens einer Schneideposition entlang einer ersten Schnittfläche durchtrennbar ist. Das Durchtrennen erfolgt dabei durch eine Abscherbewegung, bei welcher die erste Schneidekante und die zweite Schneidekante in hinreichend kleinem Abstand aneinander vorbei bewegt werden.

Weiterhin umfasst das Schneidewerkzeug mindestens einen Niederhalter, welcher derart ausgebildet und angeordnet ist, dass beim Durchtrennen der Knochenplatte eine Verbiegung der Knochenplatte durch einen Kontakt des Niederhalters mit der Knochenplatte im Wesentlichen verhindert wird. Insbesondere kann der Niederhalter derart ausgebildet und angeordnet sein, dass eine Verbiegung im Bereich der Schnittfläche verhindert wird.

Erfindungsgemäss ist der Niederhalter derart ausgebildet und angeordnet, dass die Knochenplatte in einer Einführrichtung im Wesentlichen geradlinig in die Schneideposition bringbar ist. Die Knochenplatte kann also durch eine im Wesentlichen geradlinige Bewegung in eine Position gebracht werden, in welcher sie mittels der ersten Schneidekante und der zweiten Schneidekante durchtrennbar ist. Die geradlinige Einführbarkeit schliesst hier und im Folgenden nicht aus, dass die Knochenplatte alternativ auch in nicht geradliniger Weise oder in zwei verschiedenen Richtungen geradlinig einführbar ist; es muss im Rahmen der Erfindung aber möglich sein, die Knochenplatte in zumindest einer Art und Weise im Wesentlichen geradlinig einführen zu können.

Insbesondere schliesst eine solche geradlinige Bewegung eine zeitgleich oder nacheinander erfolgende Verschiebung und Verschwenkung der Knochenplatte aus, wie sie der in DE 43 08 319 gezeigte Haltestift zwingend erforderlich macht. Das Einführen der Knochenplatte wird durch die geradlinige Einführbarkeit wesentlich vereinfacht.

Weiterhin bedingt die im Wesentlichen geradlinige Einführbarkeit in der Einführrichtung, dass die Knochenplatte in dieser Einführrichtung verschiebbar ist. Folglich ist es möglich, die Knochenplatte entlang der Einführrichtung in verschiedene Schneidepositionen relativ zur ersten Schneidekante und zur zweiten Schneidekante zu bringen, welche jeweils eine verschiedene Schnittfläche der Knochenplatte definieren. Dies erlaubt ein sehr individuelles Durchtrennen einer vorgegebenen Knochenplatte.

Zudem impliziert die im Wesentlichen geradlinige Einführbarkeit auch das Fehlen von Bauelementen, welche dieses geradlinige Einführen verhindern könnten, wie beispielsweise der in DE 43 08 319 vorhandene Haltestift. Die Verwendbarkeit des erfindungsgemässen Schneidewerkzeugs ist daher nicht auf Knochenplatten mit bestimmten Durchmessern der Durchgangsöffnungen oder bestimmten Abständen derselben eingeschränkt. Folglich ist das erfindungsgemässe Schneidewerkzeug wesentlich vielseitiger einsetzbar als das in DE 43 08 319 gezeigte.

Überdies werden Beschädigungen an der Innenkontur der Durchgangsöffnung verhindert, die durch den Kontakt mit beispielsweise einem Haltestift entstehen könnten. Die Knochenplatte muss nur an weniger empfindlichen Teilen der Oberfläche mit dem Schneidewerkzeug in Kontakt gebracht werden. Dies ist insbesondere dann vorteilhaft, wenn die Innenkontur der Durchgangsöffnung eine wie beispielsweise in WO 2004/086990 gezeigte Verblockungsstruktur enthält.

Beim Durchtrennen der Knochenplatte werden die erste und die zweite Schneidekante aneinander vorbei bewegt. Durch einen Kontakt der Knochenplatte mit dem Niederhalter wird bewirkt, dass sich die Knochenplatte bei dieser Bewegung im Wesentlichen nicht verbiegt. Die Knochenplatte stützt sich also beim Durchtrennen am Niederhalter ab. Somit werden beispielsweise unerwünschte schräge und/oder scharfkantige Schnittflächen vermieden, die bei einer übermässigen Verbiegung entstehen könnten.

Bevorzugt sind die erste Schneidekante und die zweite Schneidekante um eine Schneidendrehachse gegeneinander drehbar oder schwenkbar. Dies ermöglicht eine besonders einfache Handhabung des Schneidewerkzeugs. Das Durchtrennen erfolgt dann mittels eines Verdrehens oder Verschwenkens der ersten Schneidekante relativ zur zweiten Schneidekante, wodurch die erste Schneidekante und die zweite Schneidekante aneinander vorbei bewegt werden. Alternativ ist es aber auch möglich und liegt im Rahmen der Erfindung, dass die zweite Schneidekante im Wesentlichen geradlinig relativ zur ersten Schneidekante bewegbar ist. Das Durchtrennen erfolgt dann mittels dieser geradlinigen Bewegung der ersten Schneidekante relativ zur zweiten Schneidekante.

Bevorzugt verläuft die Einführrichtung im Wesentlichen senkrecht zu der Schneidendrehachse. Auf diese Weise wird beispielsweise verhindert, dass Teile der Knochenplatte beim Einführen am Schneidewerkzeug, beispielsweise an der Schneidendrehachse anstossen. Ein solches Anstossen, welches beispielsweise bei Y-oder n-förmigen Knochenplatten auftreten könnte, könnte das Einführen in die Schneideposition mitunter komplett verhindern, was das Schneidewerkzeug für diese Knochenplatte unbrauchbar machen würde.

Weiterhin bevorzugt umfasst das Schneidewerkzeug eine erste Schneidebacke, welche die erste Schneidekante enthält, und eine zweite Schneidebacke, welche die zweite Schneidekante enthält. Die zweite Schneidebacke kann dabei mittels eines die Schneidendrehachse definierenden schneidseitigen Gelenks gegenüber der ersten Schneidebacke drehbar oder schwenkbar sein. Die erste Schneidekante kann dabei direkt in die erste Schneidebacke gearbeitet sein, und/oder die zweite Schneidekante kann direkt in die zweite Schneidebacke gearbeitet sein.

In bevorzugten Ausführungsformen weist die erste Schneidebacke eine Auflagefläche für die Knochenplatte auf. Diese kann derart ausgebildet und angeordnet sein, dass in der Schneideposition zumindest ein Teil der Knochenplatte in Kontakt mit der Auflagefläche ist und zwischen der Auflagefläche und den Niederhalter positioniert ist. Hierdurch kann der Teil der Knochenplatte vor und/oder beim Durchtrennen an der Auflagefläche aufliegen, was ein sichereres Positionieren ermöglicht und ein unbeabsichtigtes Verschieben der Knochenplatte während des Durchtrennens verhindert.

Dabei ist es besonders vorteilhaft, wenn die Auflagefläche im Wesentlichen komplementär zumindest zu einem Teil der Oberfläche der Knochenplatte ist. Ein unbeabsichtigtes Verschieben der Knochenplatte beim Durchtrennen kann hierdurch wirkungsvoll vermieden werden. Im Falle einer Knochenplatte mit einer im Wesentlichen ebenen Oberfläche kann die Auflagefläche beispielsweise ebenfalls im Wesentlichen eben sein. Weist ein Teil der Oberfläche der Knochenplatte eine konvexe Krümmung auf, so kann die Auflagefläche entsprechend konkav geformt sein.

Besonders bevorzugt ist der Niederhalter mit der ersten Schneidebacke verbunden. Insbesondere kann er starr mit der ersten Schneidebacke verbunden sein. Allerdings ist es auch denkbar, dass der Niederhalter beweglich mit der ersten Schneidebacke verbunden ist und lediglich zum Durchtrennen an der ersten Schneidebacke starr fixierbar ist, beispielsweise mittels einer entsprechend ausgebildeten Rastvorrichtung. Dies kann insbesondere dann von Vorteil sein, wenn Knochenplatten mit verschiedenen Dicken durchtrennt werden sollen. Darüber hinaus ist es denkbar und liegt im Rahmen der Erfindung, dass der Niederhalter von der ersten Schneidebacke lösbar ist. Insbesondere kann er dann durch einen anderen Niederhalter ausgetauscht werden, welcher für eine bestimmte Knochenplatte besser geeignet ist. Beispielsweise können verschiedene Niederhalter verschiedene Dimensionen haben oder derart ausgebildet sein, dass sie in verschiedenen Positionen, insbesondere Abständen relativ zur Auflagefläche positionierbar sind. Beispielsweise können die verschiedenen Niederhalter verschieden geformte und/oder dimensionierte Zwischenräume zum Einführen einer Knochenplatte bilden, wie etwa gebogene oder halbrunde.

Es ist auch denkbar und liegt im Rahmen der Erfindung, dass der Niederhalter an der zweiten Schneidebacke angeordnet ist. Bevorzugt ist der Niederhalter dann derart federnd an der zweiten Schneidebacke gelagert, dass er beim Einleiten der Abscherbewegung zunächst in Kontakt mit der Knochenplatte gerät und die Knochenplatte bei der weiteren Abscherbewegung aufgrund der Federung gegen die erste Schneidebacke drückt.

Bevorzugt umfasst der Niederhalter einen Quersteg, welcher sich insbesondere im Wesentlichen senkrecht zur Einführrichtung erstrecken kann. Der Quersteg ist dabei derart ausgebildet und angeordnet, dass beim Durchtrennen der Knochenplatte eine Verbiegung der Knochenplatte durch einen Kontakt des Querstegs mit der Knochenplatte im Wesentlichen verhindert wird. Dies erlaubt eine besonders einfache Bauweise. Bevorzugt ist der Quersteg mittels mindestens eines Haltesteges an der ersten Schneidebacke befestigt. Besonders bevorzugt ist der Quersteg mittels zweiter seitlicher Haltestege an der ersten Schneidebacke befestigt. Dies ermöglicht eine Material sparende und gleichzeitig stabile Befestigung des Querstegs an der Schneidebacke.

Die Länge des Querstegs ist bevorzugt an die Breite von in der Osteosynthese üblicherweise verwendeten Knochenplatten angepasst. Demgemäss hat er bevorzugt eine Länge zwischen 1 mm und 15 mm, weiter bevorzugt zwischen 2 mm und 10 mm, besonders bevorzugt zwischen 3,2 mm und 7,5 mm.

Gemäss bevorzugten Ausführungsformen weist das Schneidewerkzeug einen ersten Griffhebel auf, welcher starr oder gelenkig mit der ersten Schneidebacke verbunden ist. Weiterhin bevorzugt weist es einen zweiten Griffhebel auf, welcher starr oder gelenkig mit der zweiten Schneidebacke verbunden ist. Durch derartige Griffhebel können auf Grund von Hebelwirkungen grosse Kräfte auf die Schneidekanten ausgeübt werden. Bevorzugt sind der erste Griffhebel und der zweite Griffhebel über ein griffseitiges Gelenk miteinander drehbar verbunden.

Viele Knochenplatten weisen Durchgangsöffnungen, wie beispielsweise Schraubenöffnungen auf, welche von in etwa ringförmigen Umrandungen umschlossen sind. Daher ist es oftmals gewünscht, die Knochenplatte an einer Schnittfläche zu durchtrennen, welche an diese Ringform angepasst ist und den Aussenrand des Ringes fortsetzt. Zu diesem Zweck sind bei bevorzugten Schneidewerkzeugen die erste Schneidekante und die zweite Schneidekante zumindest teilweise gekrümmt, insbesondere kreisbogenförmig ausgebildet. Dies erlaubt ein Durchtrennen entlang einer Schnittfläche, welche dieser Bogenform entspricht. Besonders bevorzugt sind die erste Schneidekante und die zweite Schneidekante bezogen auf die Einführrichtung der Knochenplatte nach aussen gebogen. Bevorzugt kann der Radius des kreisbogenförmigen Teils der ersten Schneidekante und/oder der zweiten Schneidekante im Bereich von 0,5 mm bis 7,5 mm, bevorzugt von 2 mm bis 6,5 mm, besonders bevorzugt von 4 mm bis 5,5 mm liegen.

Besonders vorteilhaft ist das Schneidewerkzeug derart ausgebildet, dass zumindest in der Schneideposition die erste Schneidekante und/oder die zweite Schneidekante einsehbar sind. Dies vereinfacht das korrekte Positionieren der Knochenplatte erheblich, da die Lage der aufgrund der Position der Schneidekanten zu erwartenden Schnittfläche erkennbar ist.

Beim Durchtrennen von Knochenplatten mit bekannten Schneidewerkzeugen kann es passieren, dass eines der beim Durchtrennen entstehenden Knochenplattenteile herunterfällt oder aufgrund der auftretenden Kräfte sogar unkontrolliert vom Schneidewerkzeug weggeschleudert wird, was nicht zuletzt ein Verletzungsrisiko in sich birgt. Daher ist das Schneidewerkzeug bevorzugt derart ausgebildet, dass mindestens eines der beim Durchtrennen der Knochenplatte entstehenden Knochenplattenteile vom Schneidewerkzeug haltbar, insbesondere einklemmbar ist. Insbesondere kann der Knochenplattenteil beim und nach dem Durchtrennen zwischen einer ersten Haltefläche an der ersten Schneidebacke und einer zweiten Haltefläche an der zweiten Schneidebacke haltbar oder einklemmbar sein. Der jeweils andere beim Durchtrennen entstehende Knochenplattenteil kann dabei bequem in einer Hand gehalten werden, während das Schneidewerkzeug in der anderen Hand gehalten wird.

Einige Ausführungsformen des erfindungsgemässen Schneidewerkzeugs enthalten zusätzlich eine Drahtschneidevorrichtung zum Durchtrennen eines länglichen Gegenstandes, beispielsweise eines chirurgischen Drahtes. Die Drahtschneidevorrichtung umfasst dabei eine erste Drahtschneidekante und eine zweite Drahtschneidekante. Die erste Drahtschneidekante ist gegenüber der zweiten Drahtschneidekante derart bewegbar, insbesondere drehbar oder schwenkbar, dass der längliche Gegenstand von der ersten Drahtschneidekante und der zweiten Drahtschneidekante durchtrennbar ist.

Die erste Drahtschneidekante kann insbesondere starr mit der ersten Schneidebacke verbunden sein; die zweite Drahtschneidekante kann starr mit der zweiten Schneidebacke verbunden sein. In diesem Falle ist die erste Drahtschneidekante um die Schneidendrehachse verschwenkbar. Hierdurch ist es möglich, dass durch die gleiche Verschwenkbewegung zwischen der ersten Schneidebacke und der zweiten Schneidebacke einerseits ein Durchtrennen einer Knochenplatte mittels der ersten Schneidekante und der zweiten Schneidekante und andererseits ein Durchtrennen eines länglichen Gegenstandes mittels der ersten Drahtschneidekante und der zweiten Drahtschneidekante erreicht werden kann.

Die Materialien und Dimensionen des Schneidewerkzeugs können entsprechend ihrer Verwendung zum Durchtrennen von Knochenplatten ausgewählt werden. Beispielsweise sind die Dimensionen, des Schneidewerkzeugs, insbesondere die Dimensionen der ersten und der zweiten Schneidefläche, der Auflagefläche und des Niederhalters auf die Dimensionen von Knochenplatten abgestimmt, welche in der Osteosynthese üblicherweise verwendet werden. Weiterhin sind die Materialien so ausgewählt, dass sie den beim Durchtrennen der Knochenplatte auftretenden Kräften standhalten können. Soll das Schneidewerkzeug beispielsweise zum Durchtrennen einer aus Titan bestehenden Knochenplatte geeignet sein, so müssen etwa die erste und die zweite Schneidefläche eine ausreichende mechanische Festigkeit aufweisen.

Ein weiterer Aspekt der Erfindung betrifft ein Osteosynthese-Set, welches mindestens eine Knochenplatte und mindestens ein erfindungsgemässes Schneidewerkzeug enthält. Das Osteosynthese-Set kann dabei auch mindestens zwei voneinander verschiedene Knochenplatten enthalten. Die mindestens eine Knochenplatte des Osteosynthese-Sets ist dabei so ausgebildet, dass sie mit Hilfe des Schneidewerkzeugs durchtrennbar ist. Insbesondere ist sie derart dimensioniert, dass sie in einer Einführrichtung im Wesentlichen geradlinig in die Schneideposition bringbar ist. Weiterhin kann beispielsweise zumindest ein Teil der Oberfläche der Knochenplatte komplementär zu der Auflagefläche des Schneidewerkzeugs sein.

Ein weiterer Aspekt der Erfindung betrifft die Verwendung eines erfindungsgemässen Schneidwerkzeugs zum Durchtrennen einer Knochenplatte.

Die Erfindung wird im Folgenden an Hand eines Ausführungsbeispiels und mehrerer Zeichnungen im Detail erläutert. Dabei zeigen
- Figuren 1a-c:: eine erfindungsgemässe Schneidezange in drei Ansichten;
- Figur 2:: einen Teil der Schneidezange in einer per- spektivischen Ansicht;
- Figuren 3a-d:: vier Detailansichten der Schneidezange ge- mäss Figuren 1a bis c mit einer Knochenplat- te in einer Schneideposition;
- Figuren 4a und b:: eine Detailansicht der Drahtschneidevorrich- tung der Schneidezange.

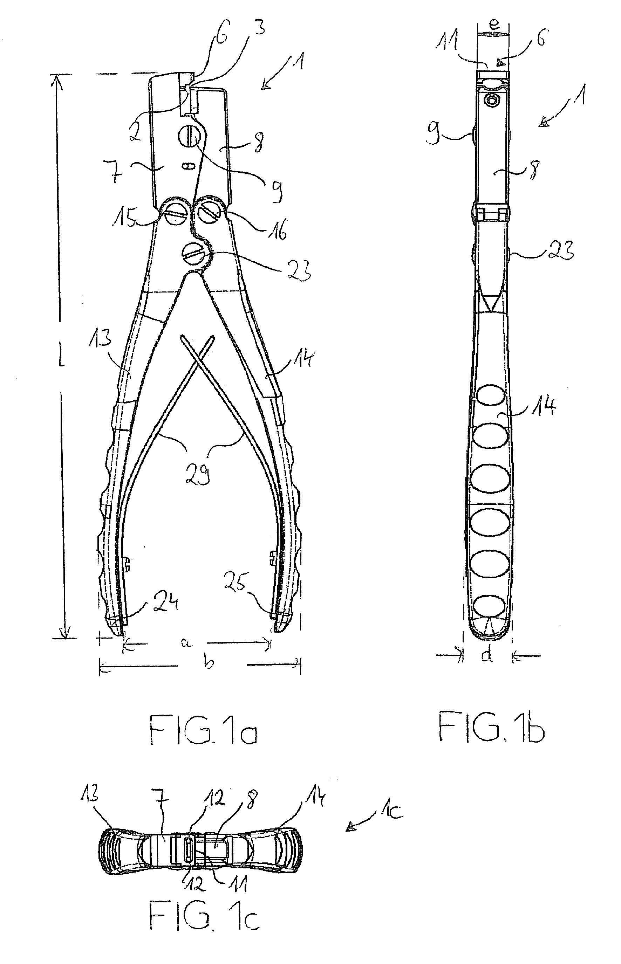

Die Figuren 1a bis 1c zeigen drei verschiedene Ansichten eines erfindungsgemässen Schneidewerkzeugs, welches als Schneidezange 1 ausgebildet ist. Gemäss der Draufsicht in Figur 1a enthält die Schneidezange 1 einen ersten Griffhebel 13, welcher über ein erstes Hebelgelenk 15 mit einer ersten Schneidebacke 7 gelenkig verbunden ist. Die Schneidzange 1 weist weiterhin einen zweiten Griffhebel 14 auf, welcher über ein zweites Hebelgelenk 16 mit einer zweiten Schneidebacke 8 gelenkig verbunden ist. Der erste Griffhebel 13 und der zweite Griffhebel 14 sind über ein griffseitiges Gelenk 23 miteinander verbunden, welches eine Schneidendrehachse definiert. Der erste Griffhebel 13 und der zweite Griffhebel 14 sind mittels zweier Federn 29 vorgespannt und wirken einem Zusammendrücken der beiden Griffhebel 13, 14 entgegen.

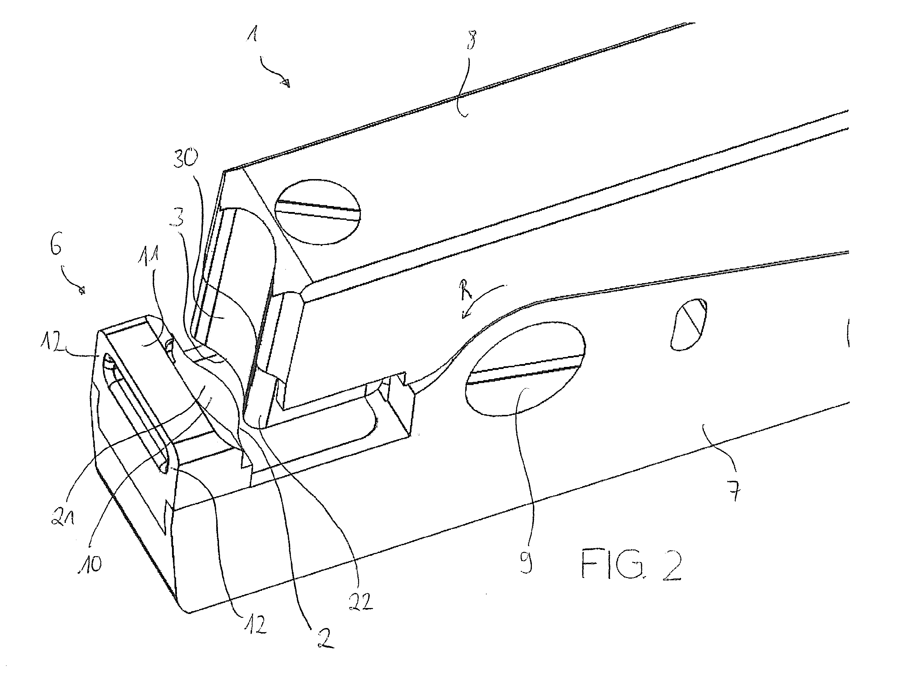

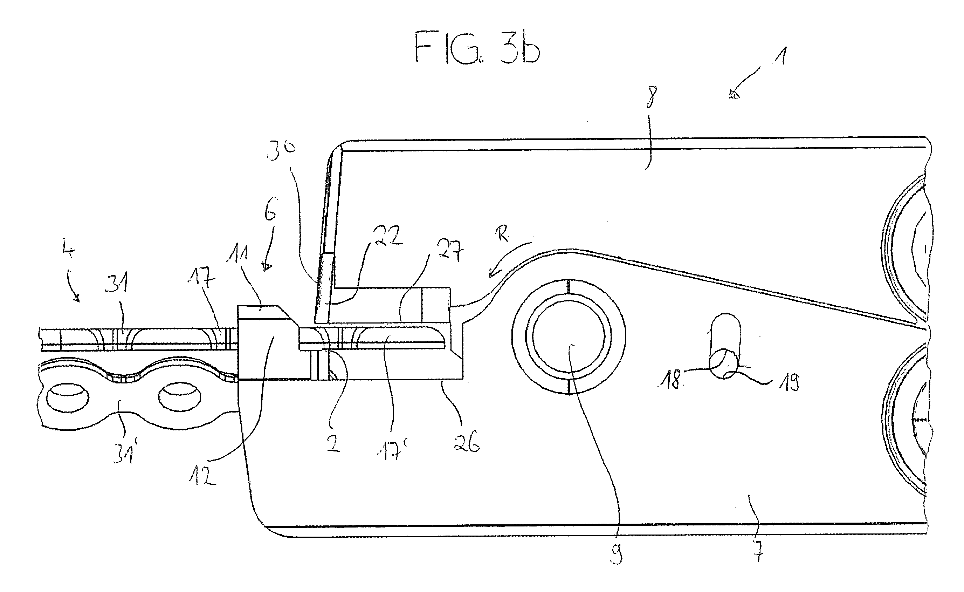

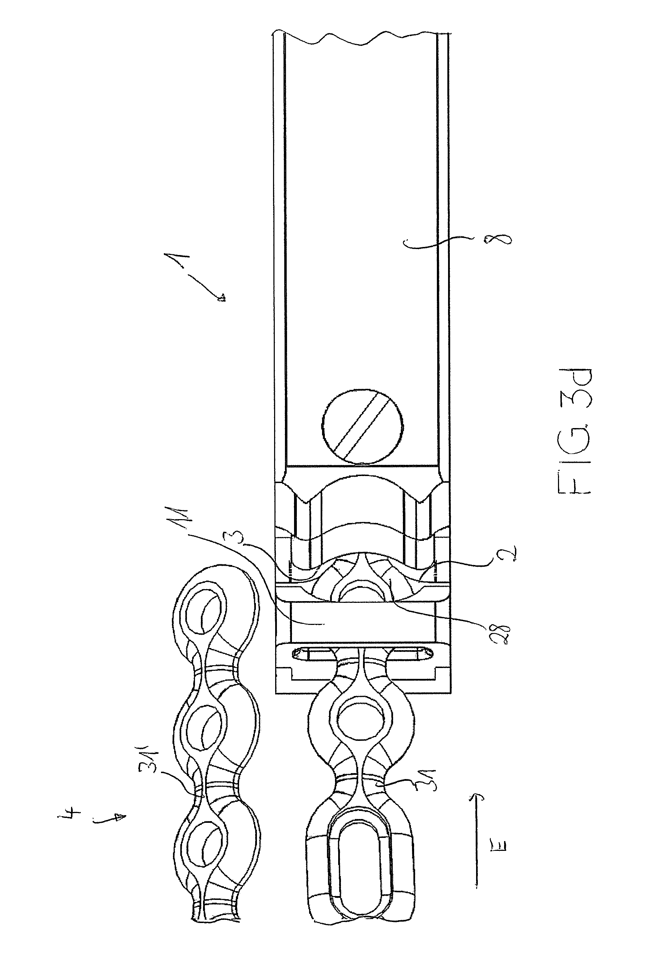

Die erste Schneidebacke 7 weist ein erstes Schneideelement 21 (siehe Figur 2) mit einer ersten Schneidekante 2 auf. Die zweite Schneidebacke 8 weist ein zweites Schneideelement 22 (siehe Figur 2) mit einer zweiten Schneidekante 3 auf. Die Vorderfläche 30 des zweiten Schneideelements 22 ist um einen Winkel von 8° zu einer Senkrechten zur zweiten Schneidebacke 8 geneigt (vgl. Figuren 3b und d). Die erste Schneidekante 2 und die zweite Schneidekante 3 sind um die durch ein schneidenseitiges Gelenk 9 definierte Schneidendrehachse gegeneinander drehbar. Die Anordnung des Gelenks 9 und weiteren Gelenken 15, 16, 23 sorgt für eine besonders geeignete Umsetzung der Kräfte. An der ersten Schneidebacke 7 ist ein Niederhalter 6 angeordnet, welcher starr mit der ersten Schneidebacke 7 verbunden ist; sein Aufbau und seine Funktion werden weiter unten detailliert erläutert.

Die Schneidezange 1 hat in der in Figur 1a dargestellten Stellung der Gelenke 9, 15, 16 und 23 eine Länge 1 über alles von 207 mm und eine Breite b über alles von 79 mm. Der erste Griffhebel 13 weist ein erstes Griffhebelende 24 auf, welches von der ersten Schneidebacke 7 abgewandt ist. Der zweite Griffhebel 14 weist ein zweites Griffhebelende 25 auf, welches von der zweiten Schneidebacke 8 abgewandt ist. Zwischen dem ersten Griffhebelende 24 und dem zweiten Griffhebelende 25 besteht in der in Figur 1a gezeigten Stellung ein Abstand a von 66 mm.

Die Figur 1b zeigt eine Seitenansicht der Schneidezange 1. Die Dicke d über alles der Schneidezange 1 im Bereich der Griffhebel 13, 14 beträgt 16 mm. Die Dicke e der ersten Schneidebacke 7 und der zweiten Schneidebacke 8 beträgt jeweils 11,4 mm. Der an der ersten Schneidebacke 7 angeordnete Niederhalter 6 verfügt über einen Quersteg 11. Wie in der Vorderansicht gemäss Figur 1c erkennbar ist, ist der Quersteg 11 mittels zweier seitlicher Haltestege 12 an der ersten Schneidebacke 7 befestigt.

In Figur 2 ist eine perspektivische Detailansicht der Schneidezange 1 dargestellt. Das erste Schneidelement 21 der ersten Schneidebacke 7 enthält eine erste Schneidekante 2; das zweite Schneideelement 22 der zweiten Schneidebacke 8 enthält eine zweite Schneidekante 3. Sowohl die erste Schneidekante 2 als auch die zweite Schneidekante 3 sind teilweise als Kreisbogen ausgebildet, wobei der Kreisbogen der zweiten Schneidekante 3 einen Radius von 4,8 mm aufweist und der Kreisbogen der ersten Schneidekante 2 einen Radius von 4,7 mm aufweist.

An der ersten Schneidebacke 7 ist weiterhin der Niederhalter 6 mit dem Quersteg 11 sichtbar. Der Quersteg 11 ist mittels zweier Haltestege 12 an der ersten Schneidebacke 7 befestigt. Das erste Schneideelement 21 der ersten Schneidebacke 7 enthält weiterhin eine Auflagefläche 10. Das dem schneidenseitigen Gelenk 9 zugewandte Ende der Auflagefläche wird von der ersten Schneidekante 2 gebildet.

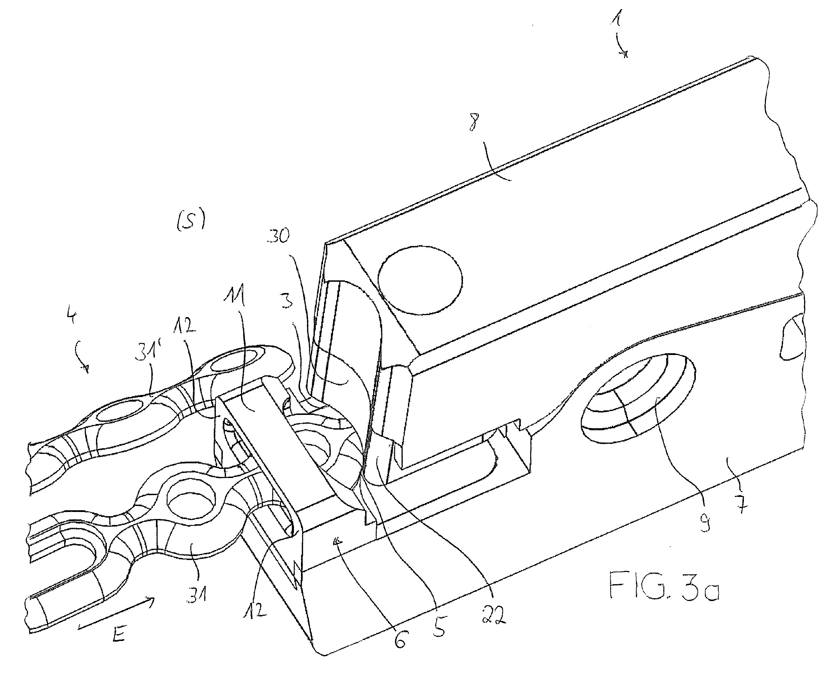

Die Figuren 3a bis d zeigen die Schneidezange 1 mit einer Knochenplatte 4 mit zwei nebeneinander angeordnete Laschen 31, 31', von denen nur die Lasche 31 durchtrennt werden soll. Die Knochenplatte 4 wurde in einer Einführrichtung E geradlinig in die in Figur 3a gezeigte Schneideposition S gebracht. Die Einführrichtung E verläuft senkrecht zur Schneidendrehachse, welche durch das schneidenseitige Gelenk 9 definiert ist. Bei dieser geradlinigen Bewegung wurde ein Teil der Lasche 31 der Knochenplatte 4 durch den Zwischenraum bewegt, welcher von dem Quersteg 11, den beiden Haltestegen 12 und der Auflagefläche 10 umschlossen ist. Die andere Lasche 31' wurde seitlich an den Schneidebacken 7 und 8 vorbeigeführt. Die geradlinige Bewegung wird dabei von keinen weiteren Bauelementen der Schneidezange 1 behindert, wie beispielsweise von Haltestiften. Die Knochenplatte 4 ist daher entlang der Einführrichtung E in einem gewissen Masse frei positionierbar. Darüber hinaus ist der Zwischenraum zwischen Quersteg 11, Haltestegen 12 und Auflagefläche 10 derart dimensioniert, dass die hier dargestellte Knochenplatte 4 auch in einem gewissen Masse drehbar ist.

Der Quersteg 11 des Niederhalters 6 erstreckt sich senkrecht zur Einführrichtung E. Die Knochenplatte 4 kann also in einer zum Quersteg 11 senkrechten Richtung eingeführt werden und ist auch in der dargestellten Schneideposition S senkrecht hierzu ausgerichtet, was eine Verbiegung der Lasche 31 der Knochenplatte 4 beim Durchtrennen besonders wirkungsvoll verhindert.

In der in Figur 3a dargestellten Schneideposition S kann die Lasche 31 der Knochenplatte 4 entlang einer Schnittfläche 5 mittels der hier nicht erkennbaren ersten Schneidekante und der zweiten Schneidekante 3 durchtrennt werden. Dies geschieht mittels eines Verschwenkens der zweiten Schneidebacke 8 gegenüber der ersten Schneidebacke 7 in der Schwenkrichtung R (siehe Figur 3b) um das schneidenseitige Gelenk 9 herum. Beim Durchtrennen entstehen im Bereich der Schnittfläche 5 Scherkräfte an der Lasche 31 der Knochenplatte 4, welche eine Tendenz zur Verbiegung der Lasche 31 hervorrufen. Hierdurch gerät die Lasche 31 in Kontakt mit der Unterseite des Querstegs 11 des Niederhalters, wodurch eine weitere Verbiegung der Lasche 31 im Bereich der Schnittfläche im Wesentlichen verhindert wird. Somit kann eine wohl definierte Schnittfläche 5 erzeugt werden, was insbesondere auch die Entstehung von scharfen Kanten verhindert.

In der Schneideposition S gemäss Figur 3a ist ein Teil der Lasche 31 der Knochenplatte 4 in Kontakt mit der Auflagefläche 10 und gegenüber vom Quersteg 11 des Niederhalters 6 positioniert. Die Auflagefläche 10 ist dabei im Wesentlichen komplementär zur Oberfläche an der Unterseite der Knochenplatte 4 ausgebildet. Ein unbeabsichtigtes Verschieben der Knochenplatte 4 beim Durchtrennen kann hierdurch wirkungsvoll vermieden werden.

Der Quersteg 11 hat eine Länge von 11,4 mm, eine in Richtung der Einführungsrichtung E gemessene Breite von 2,9 mm und eine dazu senkrechte Dicke von 1,4 mm. Die beiden Haltestege 12 haben jeweils eine in der Einführungsrichtung E gemessene Breite von 1,7 mm und eine dazu senkrechte Dicke, welche an dem der Quersteg 11 zugewandten Ende 2,9 mm und an dem dem Quersteg 11 abgewandten Ende 4,5 mm beträgt.

In Figur 3b ist ein Teil der Schneidezange 1 in einer Seitenansicht dargestellt, in welcher die Neigung der Vorderfläche 30 des zweiten Schneideelements 22 um 8° erkennbar ist. In dieser Blickrichtung sind in der dargestellten Schneideposition S sowohl die erste Schneidekante 2 als auch die zweite Schneidekante 3 einsehbar, was das korrekte Positionieren der Knochenplatte 4 im Hinblick auf die zu erzeugende Schnittfläche erheblich vereinfacht.

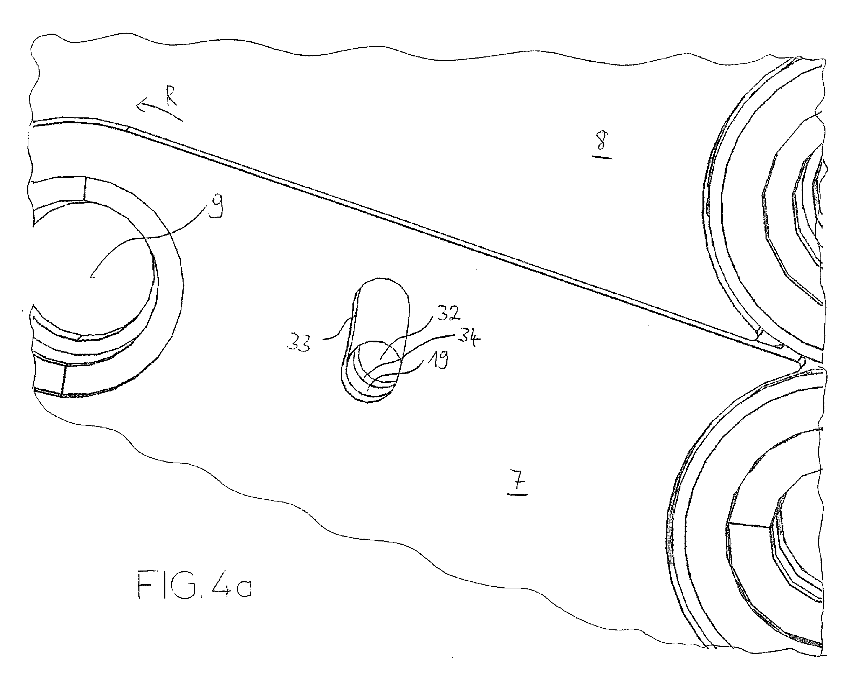

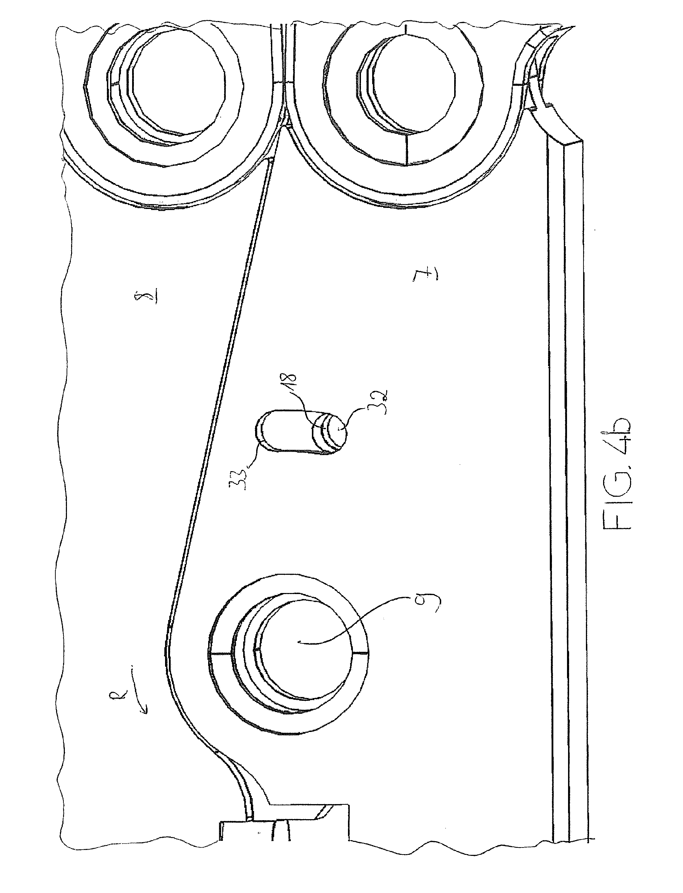

Bei einer Schwenkbewegung der zweiten Schneidebacke 8 gegenüber der ersten Schneidebacke 7 in der Schwenkrichtung R um das schneidenseitige Gelenk 9 wird die zweite Schneidekante 3 an der ersten Schneidekante 2 vorbeibewegt. Dabei wird ein erster Knochenplattenteil 17' der Lasche 31 von einem zweiten Knochenplattenteil 17 der Lasche 31 abgetrennt. Der erste Knochenplattenteil 17' wird beim Durchtrennen zwischen einer ersten Haltefläche 26 an der ersten Schneidebacke 7 und einer zweiten Haltefläche 27 an der zweiten Schneidebacke 8 eingeklemmt. Dieser Knochenplattenteil 17' kann somit beim Durchtrennen nicht herunterfallen oder gar unkontrolliert von der Schneidezange 1 weggeschleudert werden. Durch eine umgekehrte Schwenkbewegung der zweiten Schneidebacke 8 relativ zur ersten Schneidebacke 7 kann der erste Knochenplattenteil 17' wieder freigegeben werden. Weiterhin ist an der ersten Schneidebacke 7 eine erste Drahtschneidekante 18 angeordnet, und an der zweiten Schneidebacke 8 ist eine zweite Drahtschneidekante 19 angeordnet. Die erste Drahtschneidekante 18 und die zweite Drahtschneidekante 19 bilden eine Drahtschneidevorrichtung zum Durchtrennen eines länglichen Gegenstandes, beispielsweise eines chirurgischen Drahtes. Weitere bauliche Details und die Funktionsweise der Drahtschneidevorrichtung sind im Zusammenhang mit den Figuren 4a und b unten erläutert.

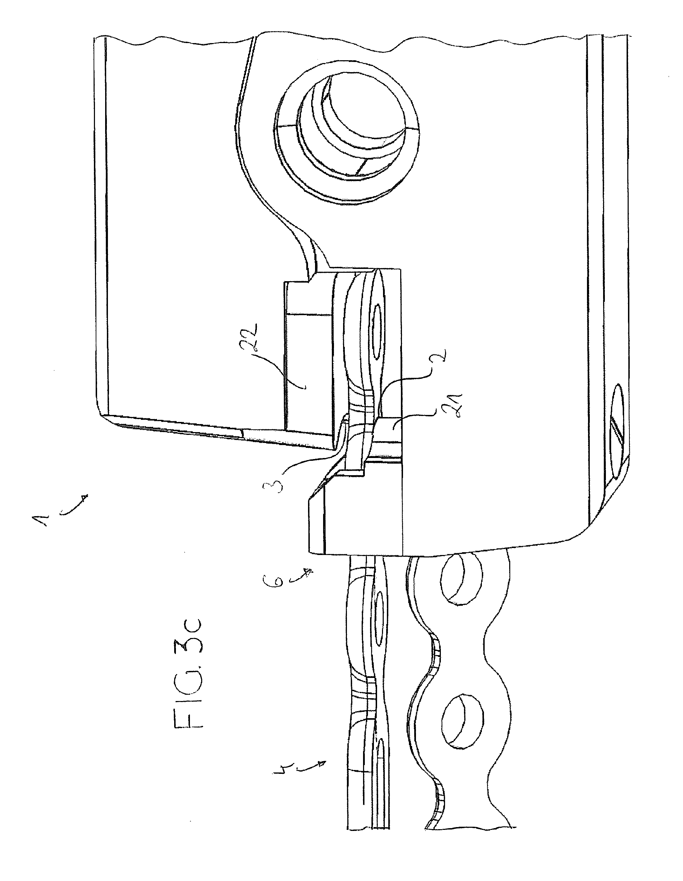

Die Figur 3c zeigt eine weitere perspektivische Ansicht der Schneidezange 1 und der Knochenplatte 4, in welcher das erste Schneideelement 21 mit der ersten Schneidekante 2 und das zweite Schneideelement 22 mit der zweiten Schneidekante 3 erkennbar sind.

In Figur 3d ist eine Draufsicht auf einen Teil der Schneidezange 1 dargestellt. Wie deutlich zu erkennen ist, ist aufgrund der Neigung der Vorderfläche 30 des zweiten Schneideelements 22 auch in dieser Blickrichtung die zweite Schneidekante 3 gut einsehbar, was das Positionieren der Knochenplatte 4 erleichtert. Die Knochenplatte 4 ist in einer derartigen Schneideposition S angeordnet, dass die zu erzeugenden Schnittfläche 5 den Aussenrand der ringförmigen Umrandung 28 eines Durchgangslochs zumindest annähernd fortsetzt.

In den Figuren 4a und b ist die Drahtschneidevorrichtung in zwei perspektivischen Darstellungen gezeigt. Gemäss der ersten Ansicht in Figur 4a weist die erste Schneidebacke 7 an ihrer Vorderseite ein bogenförmiges Langloch 33 auf. Durch das Langloch 33 hindurch sind die an der zweiten Schneidebacke 8 angeordnete zweite Drahtschneidekante 19 sowie ein kreisförmiges Loch 34 an der Rückseite der ersten Schneidebacke 7 erkennbar. Wie in Figur 4b gezeigt ist, weist das kreisförmige Loch 34 an der Rückseite der ersten Schneidebacke 7 eine erste Drahtschneidekante 18 auf, welche ebenfalls die Drahteinführungsöffnung 32 umschliesst.

Das Langloch 33 an der Vorderseite, die zweite Drahtschneidekante 19 und das kreisförmige Loch 34 an der Rückseite umschliessen eine Drahteinführungsöffnung 32.

Die erste Drahtschneidekante 18 und die zweite Drahtschneidekante 19 sind jeweils halbkreisbogenförmig ausgebildet und um das schneidenseitige Gelenk 9 herum gegeneinander verschwenkbar. Zum Durchtrennen eines länglichen Gegenstandes, beispielsweise eines chirurgischen Drahtes, wird dieser Gegenstand zunächst bis zu einer gewünschten Tiefe in die Drahteinführöffnung 32 eingeführt. Anschliessend werden der erste Griffhebel 13 und der zweite Griffhebel 14 der Schneidezange 1 aufeinander zu bewegt, so dass die zweite Schneidebacke 8 relativ zur ersten Schneidebacke 7 in der Schwenkrichtung R verschwenkt wird. Auf diese Weise wird die zweite Drahtschneidekante 19 der zweiten Schneidebacke 8 an der ersten Drahtschneidekante 18 der ersten Schneidebacke 7 vorbeigeführt, wodurch der Gegenstand an diesen Kanten abgeschert wird.

Der erste Griffhebel 13, der zweite Griffhebel 14, die erste Schneidebacke 7 und die zweite Schneidebacke 8 bestehen aus gehärtetem Stahl, bevorzugt Stahl der Werkstoffnummer 1.4021. Diese Bauteile können beispielsweise durch Schmieden und Fräsen hergestellt werden. Der Niederhalter 6 und die Auflagefläche 10 bestehen aus gehärtetem Stahl, bevorzugt Stahl der Werkstoffnummer 1.4112 und können etwa durch Fräsen hergestellt werden. Die erste Schneidekante 2 und die zweite Schneidekante 3 bestehen aus gehärtetem Stahl, bevorzugt Stahl der Werkstoffnummer 1.4112.

## Patentansprüche

1. Schneidewerkzeug zum Durchtrennen von Knochenplatten (4), insbesondere Schneidezange (1), enthaltend
- mindestens eine erste Schneidekante (2) und mindestens eine zweite Schneidekante (3), mittels welcher die Knochenplatte (4) in mindestens einer Schneideposition (S) entlang einer Schnittfläche (5) durchtrennbar ist;
- mindestens einen Niederhalter (6), welcher derart ausgebildet und angeordnet ist, dass beim Durchtrennen der Knochenplatte (4) eine Verbiegung der Knochenplatte (4), insbesondere im Bereich der Schnittfläche (5), durch einen Kontakt des Niederhalters (6) mit der Knochenplatte (4) im Wesentlichen verhindert wird;
**dadurch gekennzeichnet, dass**
der Niederhalter (6) derart ausgebildet und angeordnet ist, dass die Knochenplatte (4) in einer Einführrichtung (E) im Wesentlichen geradlinig in die Schneideposition (S) bringbar ist.

2. Schneidewerkzeug gemäss Anspruch 1,
**dadurch gekennzeichnet, dass**
die erste Schneidekante (2) und die zweite Schneidekante (3) um eine Schneidendrehachse gegeneinander drehbar oder schwenkbar sind.

3. Schneidewerkzeug gemäss Anspruch 2,
**dadurch gekennzeichnet, dass**
die Einführrichtung (E) im Wesentlichen senkrecht zur Schneidendrehachse verläuft.

4. Schneidewerkzeug gemäss einem der Ansprüche 2 und 3,
**gekennzeichnet durch**
- eine erste Schneidebacke (7), welche die erste Schneidekante (2) enthält, und
- eine zweite Schneidebacke (8), welche die zweite Schneidekante (3) enthält und mittels eines die Schneidendrehachse definierenden schneidenseitigen Gelenks (9) gegenüber der ersten Schneidebacke (7) drehbar oder schwenkbar ist.

5. Schneidewerkzeug gemäss Anspruch 4,
**dadurch gekennzeichnet, dass**
die erste Schneidebacke (7) eine Auflagefläche (10) aufweist, welche derart ausgebildet und angeordnet ist, dass in der Schneideposition (S) zumindest ein Teil der Knochenplatte (4) in Kontakt mit der Auflagefläche (10) ist und zwischen der Auflagefläche (10) und dem Niederhalter (6) positioniert ist.

6. Schneidewerkzeug gemäss Anspruch 5,
**dadurch gekennzeichnet, dass**
die Auflagefläche (10) im Wesentlichen komplementär zu zumindest einem Teil der Oberfläche der Knochenplatte (4) ist.

7. Schneidewerkzeug gemäss einem der Ansprüche 4 bis 6,
**dadurch gekennzeichnet, dass**
der Niederhalter (6) insbesondere starr mit der ersten Schneidebacke (7) verbunden ist.

8. Schneidewerkzeug gemäss einem der Ansprüche 1 bis 7,
**dadurch gekennzeichnet, dass**
der Niederhalter (6) einen Quersteg (11) umfasst, welcher sich insbesondere im Wesentlichen senkrecht zur Einführrichtung (E) erstreckt.

9. Schneidewerkzeug gemäss Anspruch 8,
**dadurch gekennzeichnet, dass**
der Quersteg (11) mittels mindestens eines Haltesteges (12), insbesondere mittels zweier seitlicher Haltestege (12) an der ersten Schneidebacke (7) befestigt ist.

10. Schneidewerkzeug gemäss einem der Ansprüche 4 bis 9,
**gekennzeichnet durch**
- einen ersten Griffhebel (13), welcher starr oder gelenkig mit der ersten Schneidebacke (7) verbunden ist;
- einen zweiten Griffhebel (14), welcher starr oder gelenkig mit der zweiten Schneidebacke (8) verbunden ist.

11. Schneidewerkzeug gemäss einem der Ansprüche 1 bis 10,
**dadurch gekennzeichnet, dass**
die erste Schneidekante (2) und die zweite Schneidekante (3) zumindest teilweise bogenförmig, insbesondere kreisbogenförmig sind.

12. Schneidewerkzeug gemäss einem der Ansprüche 1 bis 11,
**dadurch gekennzeichnet, dass**
es derart ausgebildet ist, dass in der Schneideposition (S) die erste Schneidekante (2) und/oder die zweite Schneidekante (3) einsehbar ist, dass insbesondere eine Vorderfläche (30) der zweiten Schneidebacke (8) unter einem Winkel angeordnet ist.

13. Schneidewerkzeug gemäss einem der Ansprüche 1 bis 12,
**dadurch gekennzeichnet, dass**
es derart ausgebildet ist, dass mindestens eines der beim Durchtrennen der Knochenplatte (4) entstehenden Knochenplattenteile (17,17') vom Schneidewerkzeug haltbar, insbesondere einklemmbar ist, insbesondere zwischen einer ersten Haltefläche (26) der ersten Schneidebacke (7) und einer zweiten Haltefläche (27) an der zweiten Schneidebacke (8).

14. Schneidewerkzeug gemäss einem der Ansprüche 1 bis 13,
**gekennzeichnet durch**
eine Drahtschneidevorrichtung zum Durchtrennen eines länglichen Gegenstandes, insbesondere eines chirurgischen Drahtes (20), wobei die Drahtschneidevorrichtung umfasst:
- eine erste Drahtschneidekante (18), welche insbesondere starr mit der ersten Schneidebacke (7) verbunden ist;
- eine zweite Drahtschneidekante (19), welche gegenüber der ersten Drahtschneidekante (18) derart bewegbar, insbesondere drehbar oder schwenkbar ist, insbesondere starr mit der zweiten Schneidebacke (8) verbunden ist, dass der längliche Gegenstand von der ersten Drahtschneidekante (18) und der zweiten Drahtschneidekante (19) durchtrennbar ist.

15. Osteosynthese-Set, enthaltend
- mindestens eine Knochenplatte (4), insbesondere mindestens zwei voneinander verschiedene Knochenplatten (4) und
- mindestens ein Schneidewerkzeug gemäss einem der Ansprüche 1 bis 14 zum Durchtrennen der Knochenplatte (4) .

16. Verwendung eines Schneidewerkzeugs (1) gemäss einem der Ansprüche 1 bis 14 zum Durchtrennen einer Knochenplatte (4).
